(19) Europäisches Patentamt
European Patent Office
Office européen des brevets

(11) Publication number : **0 461 815 A1**

(12) # EUROPEAN PATENT APPLICATION

(21) Application number : **91305147.0**

(51) Int. Cl.$^5$ : **C07H 19/09**, A61K 31/70

(22) Date of filing : **07.06.91**

(30) Priority : **09.06.90 GB 9012899**

(43) Date of publication of application :
**18.12.91 Bulletin 91/51**

(84) Designated Contracting States :
**AT BE CH DE DK ES FR GB GR IT LI LU NL SE**

(71) Applicant : **THE WELLCOME FOUNDATION LIMITED**
**Unicorn House 160 Euston Road**
**London NW1 2BP (GB)**

(72) Inventor : **Purifoy, Dorothy Jane Martin**
**Langley Court**
**Beckneham, Kent BR3 3BS (GB)**

(74) Representative : **Garrett, Michael et al**
**The Wellcome Research Laboratories Group Patents and Agreements Langley Court**
**Beckenham Kent BR3 3BS (GB)**

(54) **Anti-HBV pyrimidine nucleoside.**

(57) This invention relates to 1-(β-D-Arabinofuranosyl)5-prop-1-ynyluracil and pharmaceutically acceptable derivatives thereof for use in the treatment of hepatitis viral infections, particularly hepatitis B viral infections.

EP 0 461 815 A1

Jouve, 18, rue Saint-Denis, 75001 PARIS

The present invention relates to a 5-propynyl pyrimidine nucleoside and physiologically acceptable esters, salts or salts of such esters thereof, for use in the treatment of hepatitis viral infections.

World wide, hepatitis B Virus (HBV) is a viral pathogen of major consequence. It is most common in Asian countries, and prevalent in sub-Saharan Africa. The virus is aetiologically associated with primary hepatocellular carcinoma and is thought to cause 80% of the world's liver cancer. In the United States more than ten thousand people are hospitalised for HBV illness each year, an average of 250 die with fulminant disease. The United States currently contains an estimated pool of 500,000 to 1-million infectious carriers. Chronic active hepatitis will develop in over 25% of carriers, and often progresses to cirrhosis. It is estimated that 5000 people die from HBV related cirrhosis each year in the USA, and that perhaps 1000 die from HBV-related liver cancer. Even when a universal HBV vaccine is in place, the need for effective anti-HBV compounds will continue. The large reservoir of persistently infected carriers, estimated at 220 million worldwide, will receive no benefit from vaccination and will continue at high risk of HBV induced liver disease. This carrier population serves as the source of infection of susceptible individuals perpetuating the instance of disease particularly in endemic areas or high risk groups such as IV drug abusers and homosexuals. Thus, there is a great need for effective antiviral agents, both to control the chronic infection and reduce progression to hepatocellular carcinoma.

Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more prolonged leading to persistent chronic liver disease as outlined above. In "Viral Infections of Humans" (second edition, Ed., Evans, A.S. (1982) Plenum Publishing Corporation, New York), Chapter 12 describes in some detail, the aetiology of viral hepatitis infections.

European patent publication no. 22384A1 discloses a general class of nucleosides for the therapeutic or prophylatic treatment of infections caused by a retrovirus, including HIV, or by hepatitis B virus.

It has now been found that 1-(β-D-arabinofuranosyl)-5-prop-1-ynyluracil previously disclosed in European Patent Publication No. 0272065 for its use against certain herpes viruses, is useful for the treatment or prophylaxis of hepatitis viral infections, particularly hepatitis B infections.

One feature of the invention provides use of a compound of formula I

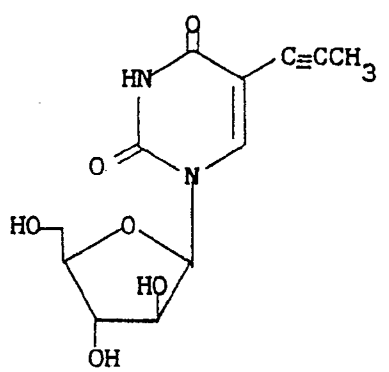

(I)

(in its enol or keto form) or a physiologically acceptable ester, salt or salt of an ester thereof, in the manufacture of a medicament for the treatment or prophylaxis of hepatitis viral infections.

In another feature is provided use of a compound of formula I above or a physiologically acceptable ester, salt or salt of such ester thereof in the manufacture of a medicament for the treatment or prophylaxis of hepatitis B viral infections.

The invention also provides a method for the treatment or prophylaxis of a mammal (particularly a human) having a hepatitis viral infection comprising administration to said mammal of an anti-hepatitis effective dose of a compound of formula I above or a physiologically acceptable ester, salt or salt of such ester.

Preferred esters of the invention include carboxylic acid esters in which the non-carbonyl moiety of the ester grouping is selected from straight or branched chain alkyl, alkoxyalkyl (e.g. methoxymethyl), carboxyalkyl (e.g. carboxyethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl-or aralkylsulphonyl (e.g. methanesulphonyl); and mono, di- or tri-phosphate esters which may or may not be blocked, amino acids esters and nitrate esters. With regard to the above-described esters, unless otherwise specified, any alkyl moieties present in such esters advantageously contain 1 to 18 carbon atoms, particularly 1 to 4 carbon atoms. Any aryl moiety present in such esters advantageously comprises a phenyl group.

The physiologically acceptable salts of the invention include base salts, for example derived from an appropriate base, such as alkali metal (e.g. sodium), alkaline earth metal (e.g. magnesium) and ammonium and $NX^{+}_{4}$ (wherein X is $C_{1-4}$ alkyl) salts.

A compound of the invention (a compound of formula I or a physiologically acceptable ester, salt or salt of such ester thereof) may be administered to a mammal including a human ("the recipient") by any route appropriate to the clinical condition to be treated; suitable routes include oral, rectal, nasal, topical (including buccal and sublingual), vaginal and parenteral (including subcutaneous, intramuscular, intravenous, intradermal, intrathecal and epidural). It will be appreciated that the preferred route may vary, for example, according to the age, weight and sex of the recipient and the nature and severity of the condition to be treated.

The amount of a compound of the invention required for the treatment of a hepatitis viral infection will depend upon a number of factors including the severity of the condition to be treated and the identity of the recipient and will ultimately be at the discretion of the attendant physician. In general, however, for each of the conditions, a suitable, effective dose will be in the range 1 to 100mg per kilogram body weight per day and most preferably in the range 5 to 30 mg per kilogram body weight per day; an optimum dose is about 15 mg per kilogram body weight per day. The effective dose may be presented as two, three, four or more sub-doses administered at appropriate intervals throughout the day. These sub-doses may be administered in unit dosage forms, for example, containing 10 to 2000 mg, preferably 20 to 500 mg and most preferably 100 to 400 mg of a compound of the invention per unit dosage form. Alternatively, if the condition of the recipient so requires, the dose may be administered as a continuous infusion.

A medicament for use in the invention preferably in the form of a pharmaceutical formulation comprising at least one compound of the invention ("the active ingredient"), together with one or more pharmaceutically acceptable carriers and optionally other therapeutic ingredients. The carrier(s) must be "acceptable" in the sense of being compatible with the other ingredients of the formulations and not deleterious to the recipients thereof.

Formulations of the invention include those suitable for administration by any of the aforementioned routes which may conveniently be presented in unit dosage form prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product. Formulations of the invention suitable for oral administration may be presented as discrete units such as capsules, cachets or tablets each containing a predetermined amount of the active ingredient; as a powder or granules; as a solution or a suspension in an aqueous liquid or a non-aqueous liquid; as an edible foam or whip; or as an oil-in-water liquid emulsion or a water-in-oil liquid emulsion. The active ingredient may also be presented as a bolus, or paste or may be contained within liposomes.

A tablet may be made by compression or moulding, optionally with one or more accessory ingredients. Compressed tablets may be prepared by compressing in a suitable machine, the active ingredient in a free-flowing form such as a powder or granules, optionally mixed with a binder (for example povidone, gelatin, hydroxypropylmethyl cellulose), lubricant, inert diluent, disintegrant (for example sodium starch glycollate, cross-linked povidone, cross-linked sodium carboxymethyl cellulose), surface-active or dispersing agent. Moulded tablets may be made by moulding in a suitable machine, a mixture of the powdered active ingredient moistened with an inert liquid diluent. The tablets are optionally coated or scored and may be formulated so as to provided slow or controlled release of the active ingredient therein, using for example, hydroxypropylmethylcellulose in varying proportions to provide the desired release profile, or to be soluble or effervescent when added to liquid.

A capsule may be made by filling a loose or compressed powder on an appropriate filling machine, optionally with one or more additives. Examples of suitable additives include binders such as povidone; gelatin, lubricants, inert diluents and disintegrants as for tablets. Capsules may also be formulated to contain pellets or discrete sub-units to provide slow or controlled release of the active ingredient. This can be achieved by extruding and spheronising a wet mixture of the drug plus an extrusion aid (for example microcrystalline cellulose) plus a diluent such as lactose. The spheroids thus produced can be coated with a semi-permeable membrane (for example ethyl cellulose, Eudragit WE30D) to produce sustained release properties.

An edible foam or whip formulation ideally comprises; 50-70% of an edible oil, particularly a vegetable oil, including corn oil, peanut oil, sunflower oil, olive oil and soybean oil; 2-10% of one or more surfactants particularly lecithin, polyols, polyol polymer esters including glyceryl fatty acid esters, polyglyceryl fatty acid esters (e.g. decaglycerol tetraoleate), or sorbitan fatty acid esters (e.g. sorbitan monostearate); 1-4% of a propellant which is suitable for ingestion, notably a compressed gas propellant especially nitrogen, nitrous oxide or carbon

dioxide, or a gaseous hydrocarbon especially propane, butane or isobutane; 0.5-30% of one or more viscosity modifiers of :>article size in the range 10-50 microns in diameter, particularly powdered sugars or colloidal silicon dioxide; and optionally 0.5-1% of one or more suitable, non-toxic colourings, flavourings or sweetners. The active ingredient is preferably present in such formulations in a concentration of 10-46%, advantageously 30%. An edible foam or whip formulation as described above may be prepared in a conventional manner, for example by mixing the edible oil, surfactant(s) and any other soluble ingredients, adding the viscosity modifier(s) aid milling the mixture to form a uniform dispersion and suspension. The active ingredient is blended into the milled mixture until evenly dispersed. Finally, a metered quantity of propellant is incorporated to the mixture after said mixture has been measured into a suitable dispensing container.

For infections of the eye or other external tissues, for example mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base or as a water-in-oil base.

If desired, the aqueous phase of the cream base may include, for example, at least 40-45% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may include a compound which enhances absorption or penetration of the active ingredient through the skin or other affected areas. Examples of such dermal penetration enhancers include dimethylsulphoxide and related analogues.

The oily phase of an emulsion formulation according to the invention may comprise merely an emulsifier (otherwise known as an emulgent), but desirably comprises a mixture of at least one emulsifier with a fat or an oil or with both a fat and an oil. Preferably, a hydrophilic emulsifier is included together with a lipophilic emulsifier which acts as a stablilizer. It is also preferred to include both an oil and a fat. Together, the emulsifer(s) with or without stabilizer(s) make up the so-called emulsifying wax, and the wax together with the oil and/or fat make up the so-called emulsifying ointment base which forms the oily phase of the cream formulations.

Emulgents and emulsion stabilizers suitable for use in the formulation of the present invention include Tween 60, Span 80, cetostearyl alcohol, myrstyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. The cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent. The ingredient is preferably present in such formulations in a concentration of 0.5 to 20%, advantageously 0.5 to 10%, particularly about 1.5% w/w.

Formulations suitable for topical administration in the mouth include lozenges comprising the active ingredient in a flavoured material, usually sucrose and acacia or tragacanth; pastilles comprising the active ingredient in an inert material such as gelatin and glycerin, or sucrose and acacia; and mouth-washes comprising the active ingredient in a suitable liquid carrier.

Formulations for rectal administration may be presented as a suppository with a suitable base comprising for example cocoa butter or higher fatty alcohol (e.g. hard wax, European Pharmacopoeia) or triglycerides and saturated fatty acids (e.g. Witepsol).

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non- aqueous sterile injection solutions which may contain anti-oxidants, buffers, bacteriostats and solutes which render the formulation isotonic

with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or sub-dose, as herein above recited, or an appropriate fraction thereof, of an active ingredient.

It should be understood that in addition to the ingredients particularly mentioned above, the formulations of this invention may include other agents conventional in the art having regard to the type of formulation in question, for example those suitable for oral administration may include flavouring agents.

A compound of the invention may be prepared by any method known in the art for the preparation of a similar compound (examples include methods described in UK Patent Specification No. 1 601 020, EP Publications Nos. 61283 and 27065, or Robins M.J. and Barr, P.J., J.Org. Chem. (1983) **48,** 1854-1862), as well as the processes described in the Examples given hereinafter.

Esters of the invention may be prepared in conventional manner for example by treatment of the compounds of formula (I) with an appropriate esterifying agent.

Salts of the invention may also be prepared in conventional manner for example by reaction of a compound of formula I with an appropriate base to form the corresponding base salt followed by acylation. Again, salts may be prepared by reaction of the compound with a base such as sodium hydride to form the corresponding sodium salt.

## Example 1

### 1-(β-D-Arabinofuranosyl)-5-prop-1-ynyluracil

#### a) $0^2$,2'-Amydrouridine

Uridine (10g, 0.04mole) was dissolved in 20ml of warm, dry dimethylformamide, and 11.4g of diphenylcarbonate (0.06m) and 0.2g of sodium bicarbonate were added. The solution was stirred and heated at 150°C until evolution of carbon dioxide ceased (30min approx). After cooling the solution was poured into 200 ml of ether with rapid stirring. The resulting solid was filtered off, washed with ether, and recrystallised from methanol to give 7.2g (80%) of the title compound, as white crystals, melting at 235-40°C.

#### b) 1-(β-D-Arabinofuranosyl)uracil

The product of Stage a) (7.0 g, 0.03mole) was dissolved in 585ml of ethanol/water (1:1) and 41ml of 1M sodium hydroxide was added. After stirring at room temperature for 2.0hr the solution was acidified to pH4-5 by portionwise addition of Dowex 50(H) ion exchange resin. The resin was filtered off and washed with 100ml of ethanol/water (1:1). The filtrate was evaporated to dryness, and the residue recrystallised from ethanol, to give 5.51g (75%) of the title compound, as white crystals, melting at 220-3°C.

#### c) 1-(β-D-Arabinofuranosyl)-5-iodouracil

The product of Stage b) (3.0 g, 12.3mmole), 3.0g of iodine (11.8mmole), 15ml of chloroform, and 30ml of 1M nitric acid were vigorously stirred and refluxed together for 2.0hr. After cooling, a crystalline solid separated, which was filtered off, and washed thoroughly with ether to remove excess iodine. The solid was recrystallised from water to give 2.55g (56%) of the title compound as white crystals melting at 191°-3°C (decomp).

#### d) 5-Iodo-1-(2,3,5-tri-O-acetyl-β-D-arabinofuranosyl)uracil

Acetic anhydride (1.04 ml, 11 mmol) was added to a solution of 1 g of 1-( -D-arabinofuranosyl)-5-iodouracil from stage c) above (2.7 mmol) in 10 ml of dry pyridine. After stirring for 3 hours at room temperature, the solvent was evaporated and the residue was co-evaporated with $CH_2Cl_2$ several times. The residue was triturated with ethanol, the solid filtered and dried to give 1.25 g (93%) of the title compound, melting at 175-9°C.

e) 5-Propynyl-1-(2,3,5-tri-O-acetyl-β-D-arabinofuranosyl)uracil

A suspension of product of stage a) (1.16 g, 2.3 mmol), 35 mg of cuprous iodide and 35 mg of bis(triphenylphosphine)palladium (II) chloride in 95 ml of dry triethylamine was stirred under dry $N_2$ for 15 mins. Propyne gas was then bubbled through the mixture for 15 mins and the mixture was stirred under an atmosphere of $N_2$ at 50°C for 1 hr. The solution was filtered and the filtrate evaporated to dryness. The residue was taken up in $CH_2Cl_2$ (30 ml) washed with 2x25 ml portions of 2% aqueous disodium ethylenediamine tetracetic acid solution and 50 ml of water. The organic solution was dried ($Na_2SO_4$) and evaporated and recrystallisation of the residue from ethanol gave 0.38 g (40%) of the title compound melting at 150-157°C.

CHN calc. C, 52.94; H, 4.902; N, 6.863%
found C, 52.86; H, 4.827; N, 6.784%

f) Product of Stage (c) above (0.3 g, 0.73 mmol) was dissolved in 20 ml of ditxan/880 ammonia/water (3:2:1) and left standing at room temperature for 18 hours. The solvent was evaporated and co-evaporated with ethanol and final recrystallisation of the residue from ethanol afforded 0.17 g of the title compound (82%) melting at 225-227°C.

CHN calc. C, 51.06; H, 4.964; N, 6.863%
found C, 50.8; H, 5.055; N, 9,8%
Mpt. - 225-227°C
'Hnmr δ($d_6$DMSO) 11.52(1H,bs,NH), 7.8(1H,s,H-6), 5.95(1H,d,H-1'), 5.65-5.0(3H,m,OH-2',OH-3',OH-5'), 4.07-3.83(2H,m,H-2',H-3'), 3.75(1H,n,H-4'), 3.59(2H,m,H-5'), 1.98ppm (3H,s,$CH_3$).

Example 2

5-Prop-1-ynyl-1-(5-O-trimethylacetyl-β-D-arabinofuranosyl)uracil

To a stirred solution of 1-(β-D-arabinofuranosyl)-5-prop-1-ynyluracil (0.28g, 1mmol, synthesised by the method described in Example 1 above in dry pyridine (5ml) at 0°C under dry nitrogen, was added dropwise a solution of trimethylacetylchloride (0.15ml, 0.14g, 1.2mmol) in dry dichloromethane (5ml) over a period of 10 minutes. The mixture was stirred at 0°C for 90 minutes then at room temperature for 2 hours. The solvent was evaporated under reduced pressure, and residual pyridine co-evaporated with portions of ethanol (3 x 25ml) to give an oil. Chromatographic separation on a silica gel column eluting with 8% methanol/dichloromethane gave pure product which was triturated with ether to give a white solid identified as the title compound.

Mpt: 204-210°C.
Analysis Calc : C-55.74, H-6.011, N-7.65%
Found: C-55.95, H-6.006, N-7.525%
δ($d_6$DMSO) 11.55(1H,6s,NH), 7.58(1H,s,H-6), 6.0(1H,d,H-1'), 5.72(1H,d,OH-2'), 5.62(1H,m,OH-3'), 4.4-4.13(2H,m,H-5'), 4.08-3.89 (3H,m,H-2',H-3',H-4'), 1.97(3H,s,C≡$CCH_3$), 1.19ppm(9H,s,tBu).

Example 3

Sodium salt of 5-Prop-1-ynyl-1-(5-trimethylacetyl-β-D-arabinofuranosyl)uracil

A suspension of sodium hydride (0.05g of 80% w/v suspension in oil, 1.66mmol, washed several times with dry tetrahydrofuran) in dry tetrahydrofuran (4ml) was added to a stirred solution of 5-prop-1-ynyl-1-(5-trimethylacetyl-β-D-arabinofuranosyl)uracil from Example 2 above (0.06g, 1.64mmol) in dry tetrahydrofuran, ensuring complete exclusion of moisture. The solvent was evaporated after 1 hour to give 0.1g of the required sodium salt.

Example A

| | Opthalmic Solution |
|---|---|
| Active ingredient | 0.5 g |
| Sodium chloride, analytical grade | 0.9 g |
| Thiomersal | 0.001 g |
| Purified water to | 100 ml |
| pH adjusted to | 7.5 |

Example B: Tablet Formulations

The following formulations A, B and C are prepared by wet granulation of the ingredients with a solution of povidone, followed by addition of magnesium stearate and compression.

Formulation A

| | | mg/tablet | mg/tablet |
|---|---|---|---|
| (a) | Active ingredient | 250 | 250 |
| (b) | Lactose B.P. | 210 | 26 |
| (c) | Povidone B.P. | 15 | 9 |
| (d) | Sodium Starch Glycollate | 20 | 12 |
| (e) | Magnesium Stearate | 5 | 3 |
| | | 500 | 300 |

Formulation B

| | | mg/tablet | mg/tablet |
|---|---|---|---|
| (a) | Active ingredient | 250 | 250 |
| (b) | Lactose | 150 | - |
| (c) | Avicel PH 101 | 60 | 26 |
| (d) | Povidone B.P. | 15 | 9 |
| (e) | Sodium Starch Glycollate | 20 | 12 |
| (f) | Magnesium Stearate | 5 | 3 |
| | | 500 | 300 |

Formulation C

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose used in formulation E is of the direct compression type.

Formulation D

| | mg/tablet |
|---|---|
| Active Ingredient | 250 |
| Pregelatinised Starch NF15 | 150 |
| | 400 |

Formulation E

| | mg/tablet |
|---|---|
| Active Ingredient | 250 |
| Lactose | 150 |
| Avicel | 100 |
| | 500 |

Formulation F (Controlled Release Formulation)

The formulation is prepared by wet granulation of the ingredients (below) with a solution of povidone followed by the addition of magnesium stearate and compression.

| | | mg/tablet |
|---|---|---|
| (a) | Active Ingredient | 500 |
| (b) | Hydroxypropylmethylcellulose (Methocel K4M Premium) | 112 |
| (c) | Lactose B.P. | 53 |
| (d) | Povidone B.P.C. | 28 |
| (e) | Magnesium Stearate | 7 |
| | | 700 |

Drug release takes place over a period of about 6-8 hours and was complete after 12 hours.

Example C: Capsule Formulations

Formulation A

A capsule formulation is prepared by admixing the uncompressed ingredients of formulation D in Example B above and filling into a two-part hard gelatin capsule. Formulation B **(infra)** is prepared in a similar manner.

Formulation B

| | | mg/capsule |
|---|---|---|
| (a) | Active ingredient | 250 |
| (b) | Lactose B.P. | 143 |
| (c) | Sodium Starch Glycollate | 25 |
| (d) | Magnesium Stearate | 2 |
| | | 420 |

Formulation C

| | | mg/capsule |
|---|---|---|
| (a) | Active ingredient | 250 |
| (b) | Macrogol 4000 BP | 350 |
| | | 600 |

Capsules are prepared by melting the Macrogol 4000 BP, dispersing the active ingredient in the melt and filling the melt into a two-part hard gelatin capsule.

Formulation D

| | mg/capsule |
|---|---|
| Active ingredient | 250 |
| Lecithin | 100 |
| Arachis Oil | 100 |
| | 450 |

Capsules are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients (a), (b), and (c) below, using an extruder, followed by spheronisation of the extrudate and drying. The dried pellets are then coated with release- controlling membrane (d) and filled into a two-piece, hard gelatin capsule.

| | | mg/capsule |
|---|---|---|
| (a) | Active Ingredient | 250 |
| (b) | Microcrystalline Cellulose | 125 |
| (c) | Lactose BP | 125 |
| (d) | Ethyl Cellulose | 13 |
| | | 513 |

Injectable Formulation

Example D:

| | |
|---|---|
| Active ingredient | 0.200 g |
| Sterile, pyrogen free phosphate buffer (pH 7.0) to | 10 ml |

The active ingredient is dissolved in most of the phosphate buffer (35- 40°C), then made up to volume and filtered through a sterile micropore filter into a sterile 10ml amber glass vial (type 1) and sealed with sterile closures and overseals.

Example E : Intramuscular Injection

| | | |
|---|---|---|
| Active Ingredient | | 0.20 g |
| Benzyl Alcohol | | 0.10 g |
| Glycofurol 75 | | 1.45 g |
| Water for Injection | q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (type 1).

Example F : Syrup Suspension

| | | |
|---|---|---|
| Active ingredient | | 0.2500 g |
| Sorbitol Solution | | 1.5000 g |
| Glycerol | | 2.0000 g |
| Dispersible Cellulose | | 0.0750 g |
| Sodium Benzoate | | 0.0050 g |
| Flavour, Peach 17.42.3169 | | 0.0125 ml |
| Purified Water | q.s. to | 5.0000 ml |

The sodium benzoate is dissolved in a portion of the purified water and the sorbitol solution added. The active ingredient is added and dispersed. In the glycerol is dispersed the thickener (dispersible cellulose). The two dispersions are mixed and made up to the required volume with tie purified water.

Example H : Suppository

| | mg/suppository |
|---|---|
| Active Ingredient (63μm) | 250 |
| Hard Fat, BP (Witepsol H15 - Dynamit NoBel) | 1770 |
| | 2020 |

* The active ingredient is used as a powder wherein at least 90% of the particles are of 63μm diameter or less.

One-fifth of the Witepsol H15 is melted in a steam-jacketed pan at 45°C maximum. The active ingredient is sifted through a 200μm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250μm stainless steel screen and, with continuous stirring, is allowed to cool to 40°C. At a temperature of 38°C to 40°C 2.02g of the mixture is filled into suitable plastic moulds. The suppositories are allowed to cool to room temperature.

Example H : Pessaries

| | mg/pessary |
|---|---|
| Active ingredient 63μm | 250 |
| Anhydrous Dextrose | 380 |
| Potato Starch | 363 |
| Magnesium Stearate | 7 |
| | 1000 |

The above ingredients are mixed directly and pessaries prepared by direct compression of the resulting mixture.

Example I : Topical formulation

| | | Cream |
|---|---|---|
| Active compound | | 5.00g |
| Glycerol | | 2.00g |
| Cetostearyl alcohol | | 6.75g |
| Sodium lauryl sulphate | | 0.75g |
| White soft paraffin | | 12.50g |
| Liquid paraffin | | 5.00g |
| Chlorocresol | | 0.10g |
| Purified water | to | 100.00g |

The active compound is dissolved in a mixture of purified water and glycerol and heated to 70°C. The remaining ingredients are heated together at 70°C. The two parts are added together and emulsified. Cooled and filled into containers.

Antiviral Activity

The human HBV producer cell line of HepG2, 2.2.15, described and characterized by Sells et al., PNAS 84:1005, 1987 and J. Virol. 62:2836, 1988, has been shown to share many characteristics of the HBV chronically infected hepatocyte. It is infectious as demonstrated by the ability to cause disease in chimpanzees. This cell line was utilized in vitro to identify compounds with anti-HBV activity.

Monolayer cultures were treated with 1-100μM of the active compound 1-(β-D-arabinofuranosyl)-5-prop-1-ynyluracil, for ten days. Supernatant media containing extracellular virion DNA (Dane particles) were harvested on day ten, treated with proteinase K (1 mg/mL) and sodium dodecyl sulfate (1%), and incubated at 50°C for one hour. DNA was extracted with equal volumes of phenol followed by chloroform and then precipitated by ammonium acetate and propanol. The DNA precipitate was dissolved and collected on nitrocellulose using the procedure of Schleicher and Schuell (S & S, 10 Optical Ave., Keene, NH 03431, Publication #700, 1987), and treated as described by Southern, J. Mol. Biol. 98:503, 1975. Cells were harvested, and the intracellular DNA. was obtained after cell lysis with guanidine isothiocyanate. The intracellular DNA was handled in the same manner as the extracellular DNA. After precipitation by ammonium acetate and propanol, the intracellular DNA precipitate was dissolved, cut by restriction endonuclease, Hind III, applied to agarose gel and then treated as described by Southern to determine the quantity of replicative intermediate forms. The antiviral effect of the drug was determined by measuring a reduction of the amount of Dane particles extruded into the culture medium (extracellular DNA) and a similar decrease in tie intracellular replicative intermediates (intracellular replicative form).

**TABLE 1**

Percent (Reduction) Inhibition of Hepatitis B Virus DNA at 100μM 1-(β-D-arabinofuranosyl)-5-prop-1-ynyluracil (compound of formula I)

| **Expt.No.** | **Released into Extracellular Media** | **Intracellular Replicative Form DNA** |
|---|---|---|
| Expt. 1 | 95.1% | 87.9% |
| Expt. 2 | 92.4% | 92.2% |
| Expt. 3 | 87.6% | 79.1% |
| Expt. 4 | 86.5% | 75.5% |

**TABLE 2**

Percent (Reduction) Inhibition of Hepatitus B Virus DNA in Extracellular Media

| Concentration of 1-(β-D-arabino furanosyl)-5-prop-1-ynynluracil | Expt. 5 | Expt. 6 |
|---|---|---|
| 100μM | 94.4<br>(88) | 98.5<br>(95.3) |
| 33μM | 87.4 | 89.1 |
| 11μM | N.I. | 71.5 |
| 37μM | 55.3 | N.I. |

N.I. = no inhibition detected
In parentheses is % reduction in intracellular hepatitis B virus DNA replicative form.

**TABLE 3**

Percent Inhibition of Hepatitis B Specific Intracellular Replicative Form (RF) DNA. Expt.7.

| Concentration of 1-(β-D-arabinofuranosyl)-5-prop-1-ynynluracil | 1μM | 10μM | 100μM |
|---|---|---|---|
| | 35% | 33% | 42% |

Cell toxicity

Cell toxicity is assessed in a cell growth inhibition assay. Subconfluent cultures of Vero cells grown on 96-well microtiter dishes are exposed to different dilutions of drug, and cell viability determined daily on replicate cultures using uptake of a tetrazolium dye (MTT). The concentration requried for a 50% inhibition of cell viability at 96 hours is termed $CCID_{50}$.

The cell toxicity for 1-(β-D-arabinofuranosyl)-5-prop-1-ynyluracil was found to be >500μM for Vero cells.

## Claims

1. Use of a compound of formula (I)

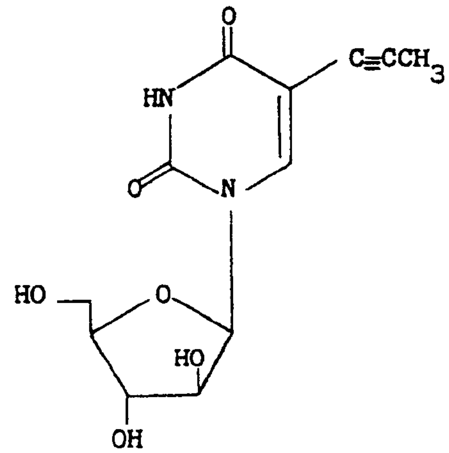

(I)

(in its enol or keto form) in the manufacture of a medicament for the treatment or prophylaxis of hepatitis viral infections.

2. Use of a physiologically acceptable ester, salt or salt of an ester of a compound of formula (I)

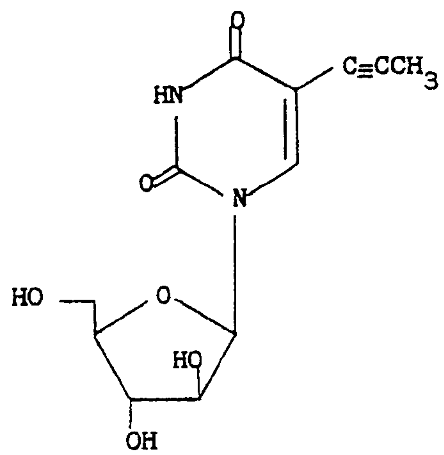

(I)

(in its enol or keto form) in the manufacture of a medicament for the treatment or prophylaxis of hepatitis viral infections.

3. Use of the compound of formula (I) according to claim 1 or a physiogically acceptable ester, salt or salt of an ester thereof according to claim 2, wherein the medicament is for the treatment or prophylaxis of hepatitis B viral infections.

4. Use of the compound of formula (I) or a physiologically acceptable ester, salt or salt of an ester thereof according to any one of claims 1 to 3 wherein the medicament is in a form suitable for oral administration.

5. Use of the compound of formula (I) or a physiologically acceptable ester, salt or salt of an ester thereof according to claim 4 wherein the medicament is in the form of a tablet or capsule.

6. Use of the compound of formula (I) or a physiologically acceptable ester, salt or salt of an ester thereof according to any one of claims 1 to 3 wherein the medicament is in a form suitable for parental administration.

7. Use of the compound of formula (I) or a physiologically acceptable ester, salt or salt of an ester thereof according to claim 6 wherein the medicament is in the form of an aqueous or non-aqueous sterile solution.

8. Use of the compound of formula (I) or a physiologically acceptable ester, salt or salt of an ester thereof according to any one of the preceding clams wherein the medicament is in a unit dosage form containing 100 to 400 mg of the compound of formula (I) or a physiologically acceptable ester, salt or salt of an ester thereof.

9. Use of the compound of formula (I) or a physiologically acceptable ester, salt or salt of an ester thereof according to any one of the preceding clams wherein the medicament is in a unit dosage form for administration to achieve an effective dose in the range of 5 to 30 mg of the compound of formula 1 or a physiologically acceptable ester, salt or salt of an ester thereof per kilogram of body weight of recipient per day.

**Claims for the following Contracting States : ES**

1. A process for preparing a pharmaceutical composition for the treatment or prophylaxis of hepatitis viral infections, containing a compound of

formula (I)A

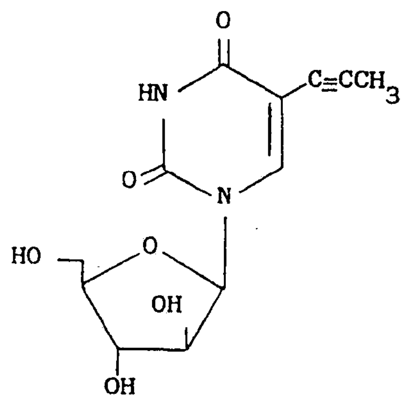

(I)A

or a pharmaceutically acceptable derivative thereof, which comprises preparing the said compound of formula (I)A by a process comprising:

A) condensing a compound of formula (II)

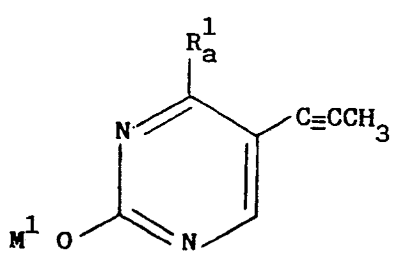

(II)

wherein $R^1_a$ represents a protected hydroxy and $M^1$ represents a hydroxy protecting group; with a compound formula (III)

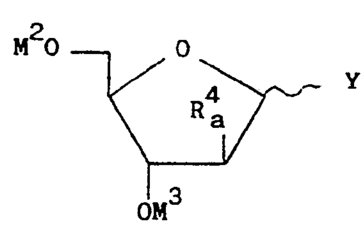

(III)

(wherein Y represents a halogen atom, $M^2$ and $M^3$ each represents a hydroxy-protecting group and $R^4_a$ represents a protected hydroxy group; or

B) reacting a compound of formula (IV)

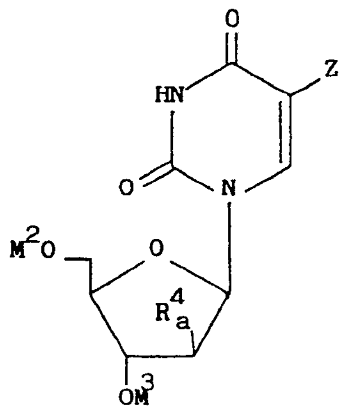

(IV)

wherein $M^2$, $M^3$ and $R^4_a$ are as defined above and Z is a leaving group, with a compound capable of providing the necessary grouping of formula - $C{\equiv}CCH_3$; and optionally thereafter or simultaneously therewith, performing either or both of the following, in any desired order:

i) removing any remaining protecting groups;

ii) where the resulting compound is a compound of formula (I)A, converting it into a pharmaceutically acceptable derivative thereof or, where the resulting compound is a pharmaceutically acceptable derivative, converting it into a different pharmaceutically acceptable derivative or a compound of formula (I)A

and bringing the resulting compound of formula (I)A or a pharmaceutically acceptable derivative thereof into association with at least one pharmaceutical excipient to form the said pharmaceutical composition.

2. A process according to claim 1 wherein the pharmaceutical composition is for the treatment of hepatitis B viral infections.

European Patent Office

# EUROPEAN SEARCH REPORT

Application Number

EP 91 30 5147

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 804 662 (ASTRA LÄKEMEDEL AB) * Abstract; page 2, lines 14-36; page 3, lines 10-21; page 4, formula IB; page 7 * | 1-9 | C 07 H 19/09<br>A 61 K 31/70 |
| Y | US-A-4 666 892 (J.J. FOX et al.) * Abstract; column 6, claim 1, lines 14-63 * | 1-9 | |
| Y | EP-A-0 356 166 (THE WELLCOME FOUNDATION LTD) * Abstract; page 2, line 1 - page 3, line 51 * | 1-9 | |
| Y | EP-A-0 322 384 (MEDIVIR AB C/O STATENS BAKTERIOLOGISKA LABORATORIUM) * Abstract; page 2, line 4 - page 6, line 3 * | 1-9 | |
| A,D | EP-A-0 272 065 (THE WELLCOME FOUNDATION LTD) * Abstract * | 1,2 | |

TECHNICAL FIELDS SEARCHED (Int. Cl.5)

C 07 H 19/00
A 61 K 31/00

The present search report has been drawn up for all claims

| Place of search | Date of completion of the search | Examiner |
|---|---|---|
| THE HAGUE | 02-10-1991 | SCOTT J.R.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)